Europäisches Patentamt

European Patent Office

Office européen des brevets

(11) Publication number: **0 498 976 B1**

## EUROPEAN PATENT SPECIFICATION

(45) Date of publication of patent specification: **05.07.95**

(51) Int. Cl.⁶: **B01J 23/84**, C07C 1/04

(21) Application number: **91301093.0**

(22) Date of filing: **12.02.91**

(54) **Method of preparation of a cobalt-rhenium catalysts for hydrocarbon synthesis.**

(43) Date of publication of application:
**19.08.92 Bulletin 92/34**

(45) Publication of the grant of the patent:
**05.07.95 Bulletin 95/27**

(84) Designated Contracting States:
**DE GB NL**

(56) References cited:
**EP-A- 0 220 343**
**EP-A- 0 266 898**

(73) Proprietor: **EXXON RESEARCH AND ENGI-
NEERING COMPANY
P.O.Box 390,
180 Park Avenue
Florham Park,
New Jersey 07932-0390 (US)**

(72) Inventor: **Mauldin, Charles Harrison
11125 Suncrest Avenue
Baton Rouge,
Louisiana 70818 (US)**

(74) Representative: **Somers, Harold Arnold et al
ESSO Engineering (Europe) Ltd.
Patents & Licences
Mailpoint 72
Esso House
Ermyn Way
Leatherhead,
Surrey KT22 8XE (GB)**

Rank Xerox (UK) Business Services
(3.10/3.09/3.3.3)

**Description**

FIELD OF THE INVENTION

This invention relates to the preparation of improved cobalt catalysts and their use in hydrocarbon synthesis processes to produce $C_5 +$ hydrocarbons from synthesis gas, carbon monoxide and hydrogen. More particularly, this invention relates to the preparation of cobalt rhenium catalysts, supported on titania, that exhibit exceedingly high volumetric productivity. These catalysts are prepared by applying cobalt and rhenium to the catalyst in incremental amounts rather than applying all of either metal in a single application.

The present invention provides a process for preparing a hydrocarbon synthesis catalyst comprising cobalt and rhenium components and a titania-comprising support, the process comprising the steps of:

(a) impregnating a titania-comprising support with either: (i) a solution containing a dissolved decomposable compound of cobalt and a solution containing a dissolved decomposable compound of rhenium, or: (ii) a solution containing dissolved decomposable compounds of cobalt and rhenium, and depositing in the support rhenium and cobalt moieties in a weight ratio of Re:Co of at least 0.05:1 and cobalt in an amount not exceeding 8 wt.%;

(b) drying the impregnated support obtained by step (a) under conditions substantially avoiding the decomposition of at least the said decomposable cobalt compound;

(c) decomposing the said decomposable compounds deposited in the dried support;

(d) repeating steps (a), (b) and (c) at least once again until the cobalt content is in the range of from 2 to 25 wt.%, based on the total weight of the composition and the weight ratio of Re to Co is at least 0.05:1.

The invention also provides a hydrocarbon synthesis process comprising passing a gas mixture containing hydrogen and carbon monoxide in a mol ratio of $H_2$:CO of at least 0.5:1 (e.g., in the range of from 0.5:1 to 4:1) in contact with a catalyst made by the process as described herein at a temperature in the range of from 160 to 290°C.

Catalysts for hydrocarbon synthesis exhibiting high volumetric activity and comprising cobalt and rhenium on a support containing primarily titania are prepared in accordance with the process of the invention, as described herein, by applying more than one application of the metals to the support. The important factors are that no more than about 8 wt% cobalt is applied in any one application cycle, and that rhenium is present during each cobalt application. After each metals application the catalyst is normally dried, followed by decomposition of the metal salts by either reduction in hydrogen or calcination with oxygen. The dispersion of the cobalt is fixed during the decomposition step. For the rhenium to function as a dispersion promoter for cobalt, it is believed, although we do not wish to be bound by any theory, that the rhenium must be present in a positive valence state when the cobalt is decomposed by converting to the elemental state or converting to the oxide.

DESCRIPTION OF THE DRAWINGS

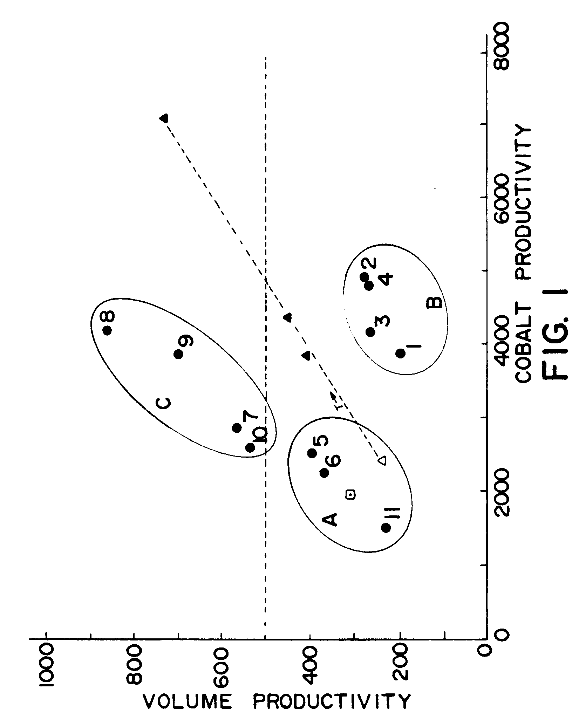

Figure 1 is a plot of volumetric productivity against cobalt productivity. The points of Group A represent the state of the art catalysts of relatively high cobalt loading, e.g., about 11-13 wt% cobalt, but relatively low cobalt productivity and relatively low volumetric productivity. The points of Group B represent catalysts having well dispersed cobalt and better cobalt productivity than the catalysts of Group A. The Group B catalysts have relatively low cobalt loadings, e.g., about 4-5 wt% cobalt. Thus, cobalt dispersion by itself is not adequate to result in high volumetric productivity. The points of Group C represent catalysts having similar cobalt loadings as Group A and better dispersion than the catalysts of A (but not as good as the dispersion of catalysts of Group B). Nevertheless, the volumetric productivity of Group C catalysts -- all of which were prepared by multiple applications of cobalt and rhenium -- is much greater than either Group A or Group B catalysts.

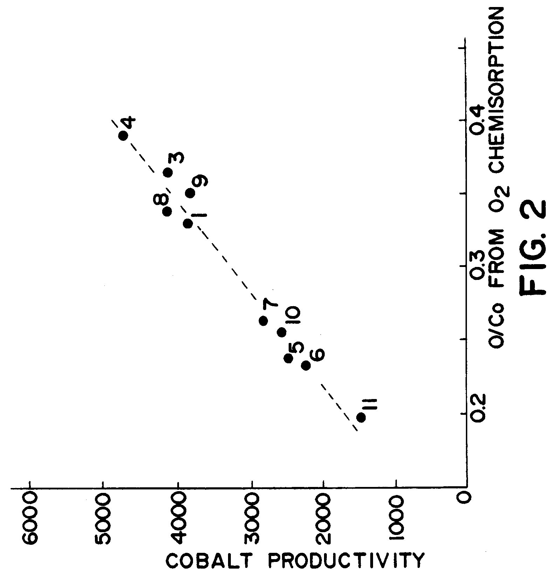

Figure 2 is a plot of cobalt productivity against cobalt dispersion measured by oxygen chemisorption, and shows that cobalt productivity increases with increasing dispersion. However, this is unrelated to volumetric activity.

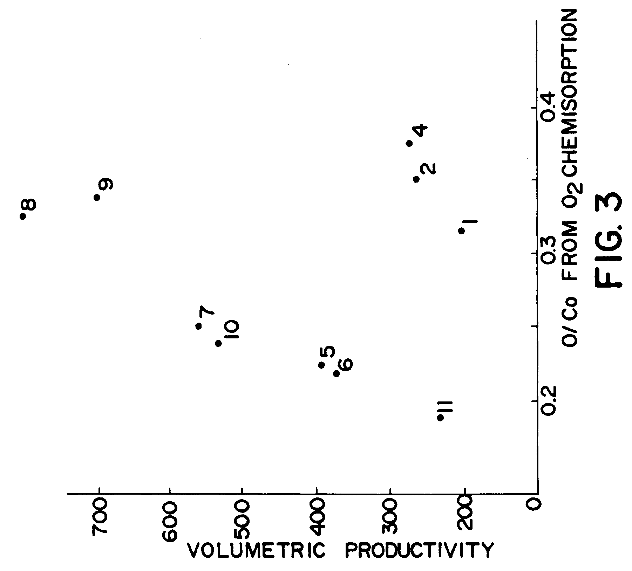

Figure 3 is a plot of volumetric productivity against cobalt dispersion similarly measured, for each of the three catalyst groups. The Group C catalysts which are indicative of this invention have high volumetric activity relative to the Group A catalysts and this difference is due to multiple applications of cobalt and rhenium to achieve greater dispersion.

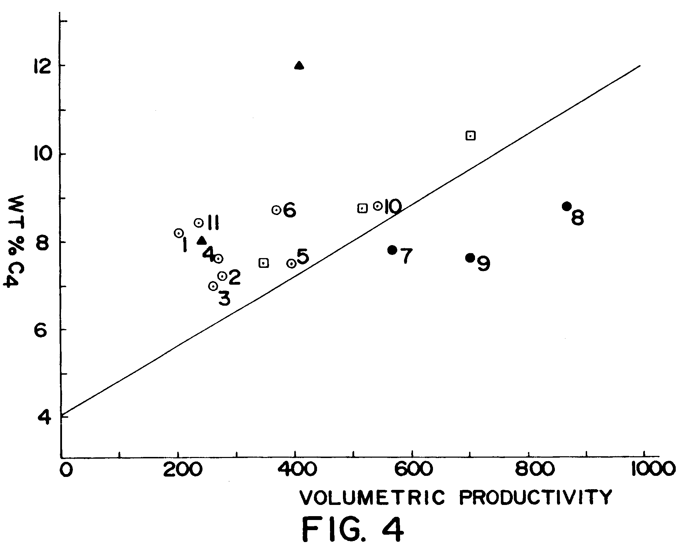

Figure 4 is a plot of volumetric productivity against weight % $C_4$-. Catalysts 7, 8, 9 which represent this invention show low selectivity to gaseous products and very high cobalt productivity. Catalysts 7, 8, and 9 are all below a line defined by the equation $C_4- = 4 + (0.008)Pv$, where Pv is volumetric productivity and $C_4-$ is wt% hydrocarbons of 4 carbons or less. Catalysts of the invention may be on this line or below it. Hence their coordinates should satisfy the relationship :

$C_4- \geq 4 + (0.008) (Pv)$.

The cobalt catalyst is one wherein cobalt is composited, or dispersed upon a suitable support, silica, alumina and preferably titania, $TiO_2$, or a titania-containing carrier, or support preferably containing more than 50 wt.% $TiO_2$. The titania preferably has a rutile:anatase weight ratio of at least about 2:3, as determined by ASTM D 3720-78: Standard Test Method for Ratio of Anatase to Rutile In Titanium Dioxide Pigments By Use of X-Ray Diffraction. In a more preferred form the titania, or titania component of the carrier, or support, when used in the conversion of synthesis gas will contain a rutile:anatase ratio of at least about 3:2, generally from about 3:2 to about 100:1, or greater, and more preferably from about 4:1 to about 100:1, or greater. The surface area of such forms of titania are less than about 50 $m^2gm^{-1}$. The cobalt is dispersed on the support in catalytically effective amounts.

In terms of absolute concentration, the cobalt is dispersed on the support in amounts ranging from about 2 percent to about 25 percent, preferably from about 5 percent to about 25 percent, more preferably about 10-25 wt%, still more preferably about 10-20 wt% based on the total weight of the catalyst composition (dry basis).

In conducting synthesis gas reactions the total pressure upon the reaction mixture is generally maintained above a gauge pressure of about 80 psig (552 kPa), and preferably above about 140 psig (965 kPa), and it is generally desirable to employ carbon monoxide, and hydrogen, in molar ratio of $H_2$:CO above about 0.5:1. Suitably, the $H_2$:CO molar ratio ranges from about 0.5:1 to about 4:1, and preferably the carbon monoxide and hydrogen are employed in molar ratio $H_2$:CO ranging from about 1:7 to about 3:1. In general, the reaction is carried out at gas hourly space velocities ranging from about 100 V/Hr/V to about 10000 V/Hr/V, preferably from about 300 V/Hr/V to about 5000 V/Hr/V, and at temperatures ranging from about 160°C to about 290°C, preferably from about 190°C to about 260°C. Pressures (gauge) preferably range from about 80 psig (552 kPa) to about 600 psig (4137 kPa), more preferably from about 140 psig (552 kPa) to about 400 psig (2758 kPa).

The catalysts employed in the practice of this invention are prepared by techniques known in the art for the preparation of these and other catalysts. Suitably, cobalt can be composited with the rhenium, upon a previously pilled, pelleted, beaded, extruded, or sieved titania or titania-containing support material by the impregnation method. In preparing catalyst, the metals are deposited from solution on the support to provide the desired absolute amount of the metals. Suitably, cobalt and rhenium are composited with the support by contacting the support with solutions of a cobalt-containing compound and rhenium containing compound or salts. The rhenium, where it is to be added, can then be composited with the support in similar manner, or the rhenium can first be impregnated upon the support, followed by impregnation of the cobalt. Optionally and preferably, the rhenium and cobalt can be coimpregnated upon the support. The cobalt compounds used in the impregnation can be any organometallic or inorganic compound which decomposes to elemental cobalt upon reduction or oxidation followed by reduction such as cobalt nitrate, acetate, acetylacetonate, naphthenate, carbonyl, or the like. Cobalt nitrate is especially preferred while cobalt halide and sulfate salts should be avoided. The salts may be dissolved in a suitable solvent, e.g., water, or organic solvent such as acetone, pentane or the like. The amount of impregnation solution used should be sufficient to completely immerse the carrier, usually within the range from about 1 to 20 times the carrier by volume, depending on the concentration of the cobalt-containing compound in the impregnation solution. The impregnation treatment can be carried out under a wide range of conditions including ambient or elevated temperatures.

Sufficient rhenium should be added to form a catalyst having a weight ratio rhenium:cobalt greater than about 0.05:1, preferably in the range of about 0.05:1 to 0.15:1 and more preferably about 0.1:1, based on the total weight of cobalt and rhenium in the catalyst (dry basis). The rhenium is deposited onto the support using a suitable compound, e.g., perrhenic acid, ammonium perrhenate, rhenium carbonyl or the like.

The catalyst, after impregnation, is dried by heating at a temperature above about 0°C but below about 125°C, in the presence of nitrogen or oxygen or both, in an air stream or under vacuum. If the salt will be decomposed by reduction, it is important that the drying conditions used do not cause premature decomposition of the impregnated cobalt salt.

The drying step can be followed either by reduction to decompose the salts or by an oxidation to convert the salts to their oxide forms. In the preferred form of this invention 10-20 wt% cobalt with concomitant amounts of rhenium are applied to the support. In order to achieve the desired volume productivity, the cobalt and rhenium are applied in more than one step with a maximum amount of cobalt of no more than about 6-8 wt% being applied in any single application. Because decomposition, by reduction or oxidation (calcination) generally sets the level of dispersion, cobalt and rhenium, applied seriatum or preferably at the same time, must be present before any decomposition step occurs.

A metals application cycle comprises, for example, impregnation with suitable metal salts followed by reduction and then followed by oxidation (calcination); or the metals impregnation may be followed directly by oxidation (calcination). A second metals application cycle may then follow the first cycle. In either case a final reduction is effected before the catalyst is charged into a hydrocarbon conversion process unit or, preferably, the final reduction takes place in the unit.

Thus, two reduction cycles wherein the metal salts are decomposed by reducing are: impregnation, reduction, oxidation; impregnation, reduction, oxidation. Additional cycles simply repeat the impregnation, reduction, oxidation cycle. And two oxidation cycles, wherein the metal salts are converted to their corresponding oxides, are: impregnation, oxidation; impregnation, oxidation. Additional cycles repeat the impregnation, oxidation cycle. A drying step may follow each impregnation and drying may occur in a reducing gas or an oxidizing gas but whether reducing or oxidizing gas is used the temperature is not such that salt decomposition or conversion to oxide, respectively, occurs.

Achieving maximum volumetric productivity (cc CO conv/hr/cc catalyst) is related to achieving maximum cobalt dispersion in any one particular cycle. The evidence of Figure 1 suggests that maximum cobalt dispersion is achieved by depositing smaller amounts of cobalt with each cycle. Thus, if a catalyst having 15 wt% cobalt is the goal, the goal may be achieved in accordance with this invention in two cycles wherein equal amounts of cobalt are deposited in each cycle. However, it is preferred to deposit the cobalt in at least three cycles wherein equal amounts of cobalt are deposited in each cycle. Of course, the cobalt may be divided into ever smaller amounts and more cycles may be employed. However, the cost of each cycle is a consideration and diminishing returns are obtained when cobalts depositions of less than about 3-4 wt% per cycle are employed. The amount of cobalt deposited in each cycle need not be equal to that in any other cycle, it is only important that cobalt deposition in any one cycle not exceed about 8 wt%. Preferably, cobalt deposition in each cycle may be in the range of 4-8 wt%, more preferably in the range of 4-6 wt%. The corresponding amount of rhenium must, of course, be present, e.g., rhenium:cobalt for each cycle of about 1:10 by weight.

Another way of determining the amount of cobalt deposited in any one cycle relates to the degree of prefilling. Thus, as pores of the carrier material are filled, the ability of the rhenium to aid in cobalt dispersion decreases, i.e., the cobalt salt tends to overwhelm the rhenium compound. The following table illustrates the degree of prefilling with a cobalt nitrate salt for two different pore volumes:

| PV, $cm^3$/gm | Wt% Co | % Pore Volume Filled By Salt |
|---|---|---|
| 0.25 | 4 | 52 |
|  | 6 | 78 |
|  | 8 | 104 |
| 0.30 | 4 | 43 |
|  | 6 | 65 |
|  | 8 | 87 |

As pore volume increases, increasing amounts of cobalt salt can be used in each cycle. Generally, pore volume filling should not exceed about 80%, preferably less than 70%, and more preferably less than about 60%. (Pore filling can be calculated in a given preparation by using density of 1.65 $gm/cm^3$ for cobalt nitrate in the molten state.) Thus, for smaller pore volumes, less cobalt per cycle should be employed.

In a reducing cycle, that is, where impregnation, for example, is followed by reduction, the salt decomposition takes place in a reducing atmosphere, preferably flowing hydrogen or a mixture of gases wherein the hydrogen component ranges in concentration from 5-100%, preferably 25-100%, at temperatures of about 200°C to about 500°C, preferably 300°C to about 450°C for a period sufficient to decompose the cobalt and rhenium salts to their metallic states. The oxidizing or calcining step whether it follows a reduction or an impregnation is conducted in flowing oxygen or a mixture of oxygen and inert gases wherein the oxygen content is at least about 1% at temperatures ranging from about 200°C to about 500°C, preferably 250°C to 450°C, for a period of time sufficient to convert the metal salts to their corresponding oxides.

EP 0 498 976 B1

In the practice of this invention the best results are achieved by multiple impregnation cycles of cobalt and rhenium wherein the impregnation step is followed by a reduction in flowing hydrogen to decompose the salts. The goal of the invention is achieving high volumetric productivity and high cobalt dispersion while noting that as the weight concentration of cobalt on the catalyst increases the cobalt dispersion and, consequently, the cobalt productivity decreases because of the increasing tendency of cobalt crystals to occupy the same site. Thus, the directional preference is for depositing smaller amounts of cobalt in each application cycle along with rhenium to aid the cobalt dispersion.

The catalyst produced by this invention has a volumetric productivity of at least about 500 (at 200°C, 2/1 $H_2$/CO) while having a cobalt dispersion, as measured by O/Co chemisorption, of at least about 0.24. Because cobalt dispersion appears directly related to cobalt productivity, the dispersion level can be equated to a cobalt productivity level of at least about 2500.

The volumetric productivity and cobalt productivity levels mentioned above are measured in a standard hydrocarbon synthesis test conducted at 200°C, a gauge pressure of 280 psig (1931 kPa) and a hydrogen:carbon monoxide mole ratio of 2. We believe that no other catalyst and particularly the catalysts reported in U.S. patent 4,499,209 and 4,568,663, can provide the volumetric productivity and cobalt dispersion of the catalysts of this invention under these conditions. However, volumetric productivity is also a function of temperature and can be increased by increasing temperature. Volumetric productivity is simply a measure of the volume of carbon monoxide converted per hour per volume of catalyst. In hydrocarbon synthesis reactions the most valuable products are $C_5$ + and preferably $C_{10}$ +. Methane is not a valuable product and, in fact, is a debit in commercial processes. Other light gases, i.e., $C_4$-while somewhat more valuable than methane, are not as valuable as liquid products. Increasing temperature in a hydrocarbon synthesis process where carbon monoxide and hydrogen are reacted, all other things being equal, tends to increase the methane yield. Nevertheless, at any reasonable temperature for hydrocarbon synthesis, the catalysts of this invention preferably have a selectivity to methane of less than about 8 mol%, preferably less than about 6 mol% at volume productivities greater than 500 at 200°C, $H_2$/CO of 2/1.

In the following examples, the $TiO_2$ extrudates used as supports had the following physical properties:
> 97% rutile (ASTM D 2730-78)
21-25 $m^2$/g (BET)
0.25-0.30 $cm^3$/g pore volume

Cobalt on titania catalysts, with or without rhenium, were prepared by evaporating acetone solutions of cobaltous nitrate and perrhenic acid onto portions of the $TiO_2$ extrudates and drying at 90°C in a vacuum oven. Dried catalyst was then charged to a quartz reactor tube and the nitrate salt decomposed by one of the cycles disclosed below:

| Cycle | Procedure |
|---|---|
| Oxidation | (1) Air, 250°C, 3 hours |
| Reduction I | (1) $H_2$, 450°C, GHSV of 800 for 1 hour, (cool below 50°C, flush with helium) <br> (2) 1% $O_2$ - 4% $N_2$ - 95% He at 250°C, GHSV of 1260 for 3 hours |
| Reduction II | (1) $H_2$, 300°C, GHSV of 800 for 1 hour (cool below 50°C, flush with helium) <br> (2) 1% $O_2$ - 4% $N_2$ - 95% He at 250°C, GHSV of 1260 for 3 hours |

The treatments used for each catalyst are indicated in Table 1 and 2. Catalyst 2 was not treated in the quartz reactor but was charged directly to the test reactor described below, where a final reduction is applied before reacting the catalyst with synthesis gas.

Catalysts were sized to 60-150 (Tyler) mesh (0.250-0.105 mm) and tested in a small fixed-bed reactor. The catalyst charge was diluted with an equal volume of 60-150 (Tyler) mesh (0.250-0.105 mm) $TiO_2$ (to minimize temperature gradients) and activated by reduction with hydrogen at 450°C, atmospheric pressure, for one hour. Synthesis gas with a composition of 64% $H_2$-32% CO-4% Ne was then converted over the activated catalyst at 200°C, a gauge pressure of 280 psig (1931 kPa) for a test period of at least 20 hours. Gas hourly space velocities (GHSV) shown in the tables represent the flow rate passed over the volume of catalyst, excluding the diluent, at 22°C conditions. Samples of the exit gas were periodically analyzed by gas chromatography to determine the extent of CO conversion and the selectivity to methane, expressed as the moles of $CH_4$ formed per 100 moles of CO converted. Methane selectivity was essentially constant at about 4-5% on all of the cobalt-rhenium catalysts described in Tables 1 and 2 (increasing slightly as temperature was increased). The remainder of the carbon-containing product was $C_2$ + hydrocarbons, predominately $C_2$ + linear paraffins.

5

Oxygen chemisorption was performed at 25°C by measuring the uptake of oxygen pulses from a helium carrier gas stream, passed over samples of catalyst which were reduced in hydrogen at 450°C.

The results are summarized in Table I and discussed thereafter.

TABLE 1

| CATALYST NUMBER | DECOMPOSITION PROCEDURE | NO. OF IMPREG/ DECOMP. STEPS | FINAL WT., %CO | RE:CO WT. RATIO | GHSV | % CO CONV. | MOL% $CH_4$ | WT% $C_4-$ | VOL.(1) PRODUCTIVITY | COBALT(2) PRODUCTIVITY | $O_2$ CHEMIS O/CO |
|---|---|---|---|---|---|---|---|---|---|---|---|
| 1 | OXIDATION | 1 | 4.67 | 0.1 | 850 | 74 | 4.6 | 8.2 | 201 | 3880 | 0.318 |
| 2 | REDUCTION(3) | 1 | 4.52 | 0.1 | 1000 | 86 | 3.9 | 7.2 | 275 | 4910 | - |
| 3 | REDUCTION I | 1 | 5.35 | 0.1 | 1000 | 82 | 3.8 | 7.0 | 262 | 4150 | 0.352 |
| 4 | REDUCTION II | 1 | 4.89 | 0.1 | 1000 | 84 | 4.2 | 7.6 | 269 | 4780 | 0.379 |
| 5 | OXIDATION | 1 | 12.1 | 0.1 | 1500 | 82 | 4.1 | 7.5 | 394 | 2470 | 0.226 |
| 6 | REDUCTION I | 1 | 11.7 | 0.1 | 1500 | 77 | 4.9 | 8.7 | 370 | 2230 | 0.222 |
| 7 | OXIDATION | 2 | 13.9 | 0.1 | 2250 | 78 | 4.3 | 7.8 | 562 | 2830 | 0.251 |
| 8 | REDUCTION II | 3 | 13.3 | 0.1 | 3500 | 77 | 5.0 | 8.8 | 862 | 4140 | 0.325 |
| 9 | OXIDATION | 3 | 13.3 | 0.1 | 3000 | 73 | 4.2 | 7.6 | 700 | 3850 | 0.339 |
| 10 | OXIDATION | 3 | 14.1 | 0.05 | 2500 | 67 | 5.0 | 8.8 | 536 | 2580 | 0.244 |
| 11 | OXIDATION | 3 | 13.2 | 0 | 1000 | 73 | 4.7 | 8.4 | 234 | 1460 | 0.187 |

(1) $cm^3$ CO converted/hour/$cm^3$ catalyst
(2) $cm^3$ CO converted/hour/g cobalt
(3) dried catalyst was charged to the test reactor and reduced at 450°C

Catalysts 1, 2, 3, and 4 (shown in Figure 1 as Group B) are cobalt-rhenium containing catalysts prepared in a single application of the metals. These catalysts have relatively low cobalt loadings and exhibit the highest cobalt dispersion and, therefore, the highest cobalt productivity. For these catalysts, a reduction following the metals impregnation shows somewhat better dispersion than an oxidation following impregnation.

Catalysts 5 and 6 (shown in Figure 1 in Group A) are cobalt-rhenium containing catalysts prepared in a single application of metals. These catalysts have higher cobalt loadings than the Group B catalysts but exhibit lower cobalt dispersion and low cobalt productivity although volumetric productivity is somewhat higher than the Group B catalysts.

Catalyst 11 (also in Group A) contains only cobalt (no rhenium) and the cobalt was applied in three application cycles of equal amounts of cobalt to reach a final cobalt loading of 13.2 wt%. This catalyst exhibits the lowest cobalt dispersion and lowest cobalt productivity. As compared to catalysts 5 and 6, the cobalt dispersion is lower and the dispersion aiding affect of rhenium can be seen. Thus, catalyst 11 shows that multiple impregnation cycles without rhenium does not provide any advantageous effect. Quite the contrary is true when rhenium is present. Also, there is very little difference in whether oxidation or reduction follows the impregnation, as shown by catalysts 5 and 6.

Group A in Figure 1 also includes a catalyst denoted by a square. This data point was taken from Table II of U.S. 4,568,663 and contained 12 wt% cobalt with rhenium:cobalt weight ratio of 0.042:1. This catalyst representing the best cobalt rhenium/titania state of the art catalyst performs no better than catalysts 5 and 6 and is simply representative of this type of catalyst prepared with single metals impregnation cycle.

Catalysts 7, 8, 9, and 10 (shown in Figure 1 as Group C) are cobalt-rhenium containing catalysts having essentially the same cobalt loadings as catalysts 5, 6, 11 and the square. However, these catalysts exhibit an exceedingly high volumetric productivity when compared to Group A cobalt-rhenium catalysts -- because of the multiple impregnation cycles employed.

Of Group C, Catalyst No. 10 had the lowest volumetric productivity due to its less than preferred rhenium:cobalt weight ratio and, therefore, a reduced dispersion enhancing effect.

Comparing the performance of catalysts 7, 8, and 9 shows that three impregnation cycles coupled with reduction following the deposition of the metals is the most preferred preparation technique and leads to the highest volumetric productivity, increasing cobalt dispersion, and increasing cobalt productivity (compare No. 8 and 9 with No. 7).

For comparison purposes, Table 2 shows the performance of the catalyst denoted by a square (in Group A). The cobalt-rhenium-titania catalyst of U.S.-A-4,568,663. Table 2 also shows the performance of four 25 wt% cobalt-18 wt% zirconium-silica catalysts, three of which are reported in U.S.-A-4,499,203 and one freshly prepared by the method reported for catalyst No. 16 of the '209 patent. The cobalt-zirconium containing catalysts results are reported for 200°C, 205°C, and 220°C operations. The volumetric productivity increases as the temperature is raised from 200°C to 220°C (except for the catalyst of experiment 20 of the '209 patent, but the volumetric productivity is higher at 220°C than it is at 200°C).

The performance of the cobalt-zirconium catalysts are noted in Figure 4 as triangles and volumetric productivity increases as a straight line function with increasing temperature.

Catalysts 7, 8, and 9, as shown in Figure 4, clearly illustrate the advantageous aspects of increased volumetric productivity and selectivity. The only other catalysts exhibiting volumetric productivity in the area of catalysts 7, 8, and 9 are the squares and catalyst no. 10. The squares represent catalysts of U.S. Patent 4,568,663 and catalyst no. 10 contains less than the preferred amount of rhenium. The two uppermost squares show high volume productivity levels but also exhibit relatively poor selectivity.

However, catalysts 7, 8, and 9 exhibit very good Pv and very good selectivity.

TABLE 2

| CATALYST DESCRIPTION | T, °C | GHSV | % CO CONV. | MOL% CH4 | WT% C4= | VOL. PROD. | CO PROD. |
|---|---|---|---|---|---|---|---|
| 25 Co - 18 Zr - 100 SiO$_2$ (US 4,499,209) | | | | | | | |
| Exp. 16 in '209 | 205 | 2000 | 71 | - | 20(1) | 454 | 4330 |
| Exp. 19 in '209 | 220 | 3000 | 77 | - | 34(1) | 739 | 7040 |
| Exp. 20 in '209 | 220 | 1500 | 85 | - | 12(1) | 408 | 3890 |
| Fresh Prep. of Cat 16 of '209 | 200 | 1000 | 75 | 4.3 | 8 | 240 | 2400 |
| Co-Re-TiO$_2$ (US 4,568,663), Ex. 2 | 200 | 1000 | 98 | 4.5 | ~8.1 | 314 | 1940 |
| Fresh Prep. of Ex. 2 of '663 | 200 | 1500 | 72 | 4.1 | 7.5 | 346 | 2270 |
| Fresh Prep. of Ex. 2 of '663 | 210 | 2100 | 77 | 4.9 | 8.7 | 517 | 3390 |
| Fresh Prep. of Ex. 2 of '663 | 218 | 3000 | 73 | 6.1 | 10.4 | 701 | 4590 |

(1) by difference

**Claims**

1. A process for preparing a hydrocarbon synthesis catalyst comprising cobalt and rhenium components and a titania-comprising support, the process comprising the steps of:
   (a) impregnating a titania-comprising support with either: (i) a solution containing a dissolved decomposable compound of cobalt and a solution containing a dissolved decomposable compound

8

of rhenium, or: (ii) a solution containing dissolved decomposable compounds of cobalt and rhenium, and depositing in the support rhenium and cobalt moieties in a weight ratio of Re:Co of at least 0.05:1 and cobalt in an amount not exceeding 8 wt.%;

(b) drying the impregnated support obtained by step (a) under conditions substantially avoiding the decomposition of at least the said decomposable cobalt compound;

(c) decomposing the said decomposable compounds deposited in the dried support;

(d) repeating steps (a), (b) and (c) at least once again until the cobalt content is in the range of from 2 to 25 wt.%, based on the total weight of the composition and the weight ratio of Re to Co is at least 0.05:1.

2. The process of claim 1 wherein the said compounds are decomposed: (a) by treatment with a hydrogen-containing gas; or (b) by treatment with an oxygen-containing gas; or (c) by treatment with a hydrogen-containing gas followed by treatment with an oxygen-containing gas.

3. The process of claim 1 or claim 2 wherein the solutions of dissolved cobalt and rhenium compounds are contacted with the support either at the same time or in serial fashion.

4. The process of any one of claims 1 to 3 wherein the wt.% cobalt applied in each step (a) is such that the pore volume of the support filled is less than about 80%.

5. The process of any one of claims 1 to 4 wherein steps (a), (b) and (c) are repeated until the catalyst contains from 10 to 25 wt.% cobalt.

6. The process of any one of claims 1 to 5 wherein the rutile:anatase ratio of the said titania is at least 2:3 (preferably 3:2).

7. The process of any one of claims 1 to 6 wherein the support comprises at least 50 wt.% $TiO_2$.

8. The process of any one of claims 1 to 7 wherein the cobalt deposition in each step (a) is in the range of from 4 to 6 wt.%.

9. The process of any one of claims 1 to 8 wherein the Re:Co weight ratio in the composition obtained from step (d) is in the range of from 0.05:1 to 0.15:1.

10. A hydrocarbon synthesis process comprising passing a gas mixture containing hydrogen and carbon monoxide in a mol ratio of $H_2$:CO of at least 0.5:1 (e.g., in the range of from 0.5:1 to 4:1) in contact with a catalyst made by the process of any one of claims 1 to 9 at a temperature in the range of from 160 to 290°C.

**Patentansprüche**

1. Verfahren zur Herstellung eines Kohlenwasserstoffsynthesekatalysators, der Kobalt- und Rheniumkomponenten und einen Titandioxid umfassenden Träger umfaßt, bei dem

(a) ein Titandioxid umfassender Träger mit entweder (i) einer Lösung, die eine aufgelöste zersetzbare Kobaltverbindung enthält, und einer Lösung, die eine aufgelöste zersetzbare Rheniumverbindung enthält, oder (ii) einer Lösung, die aufgelöste zersetzbare Verbindungen von Kobalt und Rhenium enthält, imprägniert wird und in den Träger Rhenium- und Kobaltanteile in einem Gewichtsverhältnis von Re:Co von mindestens 0,05:1 und Kobalt in einer Menge, die 8 Gew.% nicht überschreitet, eingebracht werden,

(b) der in Stufe (a) erhaltene imprägnierte Träger unter Bedingungen getrocknet wird, die die Zersetzung von mindestens der zersetzbaren Kobaltverbindung im wesentlichen vermeiden,

(c) die in den getrockneten Träger eingebrachten zersetzbaren Verbindungen zersetzt werden, und

(d) Stufen (a), (b) und (c) mindestens ein weiteres Mal wiederholt werden, bis der Kobaltgehalt im Bereich von 2 bis 25 Gew.% liegt, bezogen auf das Gesamtgewicht der Zusammensetzung, und das Gewichtsverhältnis von Re zu Co mindestens 0,05:1 beträgt.

2. Verfahren nach Anspruch 1, bei dem die Verbindungen durch (a) Behandlung mit einem wasserstoffhaltigen Gas, oder (b) Behandlung mit einem sauerstoffhaltigen Gas, oder (c) Behandlung mit einem wasserstoffhaltigen Gas und anschließende Behandlung mit einem sauerstoffhaltigen Gas zersetzt werden.

3. Verfahren nach Anspruch 1 oder Anspruch 2, bei dem die Lösungen der aufgelösten Kobalt- und Rheniumverbindungen entweder gleichzeitig oder aufeinanderfolgend mit dem Träger kontaktiert werden.

4. Verfahren nach einem der Ansprüche 1 bis 3, bei dem in jeder Stufe (a) soviel Gew.% Kobalt eingesetzt werden, daß das Porenvolumen des Trägers zu weniger als etwa 80 % gefüllt ist.

5. Verfahren nach einem der Ansprüche 1 bis 4, bei dem die Stufen (a), (b) und (c) wiederholt werden, bis der Katalysator 10 bis 25 Gew.% Kobalt enthält.

6. Verfahren nach einem der Ansprüche 1 bis 5, bei dem das Rutil:Anatas-Verhältnis des Titandioxids mindestens 2:3 (vorzugsweise 3:2) beträgt.

7. Verfahren nach einem der Ansprüche 1 bis 6, bei dem der Träger mindestens 50 Gew.% $TiO_2$ umfaßt.

8. Verfahren nach einem der Ansprüche 1 bis 7, bei dem die eingebrachte Kobaltmenge in jeder Stufe (a) im Bereich von 4 bis 6 Gew.% liegt.

9. Verfahren nach einem der Ansprüche 1 bis 8, bei dem das Re:Co-Gewichtsverhältnis in der aus Stufe (d) erhaltenen Zusammensetzung im Bereich von 0,05:1 bis 0,15:1 liegt.

10. Kohlenwasserstoffsyntheseverfahren, bei dem eine Wasserstoff und Kohlenmonoxid in einem Molverhältnis von $H_2$:CO von mindestens 0,5:1 (z. B. im Bereich von 0,5:1 bis 4:1) enthaltende Gasmischung bei einer Temperatur im Bereich von 160°C bis 290°C in Kontakt mit einem nach dem Verfahren gemäß einem der Ansprüche 1 bis 9 hergestellten Katalysator gebracht wird.

**Revendications**

1. Procédé de préparation d'un catalyseur de synthèse d'hydrocarbures comprenant des composants de cobalt et de rhénium et un support comprenant du dioxyde de titane, le procédé comprenant les stades suivants :
   (a) on imprègne un support comprenant du dioxyde de titane avec soit (i) une solution contenant un composé décomposable dissous de cobalt et une solution contenant un composé décomposable dissous de rhénium, soit une solution contenant des composés décomposables dissous de cobalt et de rhénium, et on dépose dans le support des radicaux de rhénium et de cobalt selon un rapport pondéral Re:Co d'au moins 0,05:1 et avec une quantité de cobalt ne dépassant pas 8 % en poids,
   (b) on sèche le support imprégné obtenu au stade (a) dans des conditions telles que l'on évite sensiblement la décomposition d'au moins ledit composé de cobalt décomposable,
   (c) on décompose lesdits composés décomposables déposés sur le support séché, et
   (d) on répète les stadés (a), (b) et (c) au moins une fois encore jusqu'à ce que la teneur en cobalt se situe dans la plage de 2 à 25 % en poids par rapport au poids total de la composition et que le rapport pondéral de Re à Co soit d'au moins 0,05:1.

2. Procédé selon la revendication 1, dans lequel lesdits composés sont décomposés (a) par traitement avec un gaz contenant de l'hydrogène ou (b) par traitement avec un gaz contenant de l'oxygène ou (c) par traitement avec un gaz contenant de l'hydrogène suivi d'un traitement avec un gaz contenant de l'oxygène.

3. Procédé selon la revendication 1 ou 2, dans lequel les solutions de composés de cobalt et de rhénium dissous sont mises en contact avec le support soit en même temps, soit à la suite.

4. Procédé selon l'une quelconque des revendications 1 à 3, dans lequel le pourcentage en poids de cobalt appliqué dans chaque stade (a) est tel que le volume de pores du support rempli soit inférieur à environ 80 %.

5. Procédé selon l'une quelconque des revendications 1 à 4, dans lequel les stades (a), (b) et (c) sont répétés jusqu'à ce que le catalyseur contienne 10 à 25 % en poids de cobalt.

6. Procédé selon l'une quelconque des revendications 1 à 5, dans lequel le rapport rutile:anatase dudit dioxyde de titane est d'au moins 2:3 (de préférence 3:2).

7. Procédé selon l'une quelconque des revendications 1 à 6, dans lequel le support comprend au moins 50 % en poids de $TiO_2$.

8. Procédé selon l'une quelconque des revendications 1 à 7, dans lequel le dépôt de cobalt dans chaque stade (a) se situe dans la plage de 4 à 6 % en poids.

9. Procédé selon l'une quelconque des revendications 1 à 8, dans lequel le rapport pondéral Re:Co de la composition obtenu au stade (d) se situe dans la plage de 0,05:1 à 0,15:1.

10. Procédé de synthèse d'hydrocarbures consistant à faire passer un mélange gazeux contenant de l'hydrogène et du monoxyde de carbone selon un rapport molaire $H_2$:CO d'au moins 0,5:1 (par exemple, dans la plage de 0,5:1 à 4:1) en contact avec un catalyseur préparé selon le procédé de l'une quelconque des revendications 1 à 9 à une température dans la plage de 160 à 290°C.

FIG. I

FIG. 2

FIG. 3

FIG. 4